# EUROPEAN PATENT SPECIFICATION

(11) **EP 4 128 275 B1**
(45) Date of publication and mention of the grant of the patent: **28.10.2026**
(21) Application number: 21719711.0
(22) Date of filing: 23.03.2021
(51) Int. Cl.: G16H 50/30, A61B 5/11, A61B 5/389, A61B 5/00

(54) **SYSTEM AND METHOD FOR CONTROLLING AN INTERACTION BETWEEN AN OPERATOR AND A MACHINE**
SYSTEM UND VERFAHREN ZUR STEUERUNG EINER INTERAKTION ZWISCHEN EINEM BEDIENER UND EINER MASCHINE
SYSTÈME ET PROCÉDÉ DE COMMANDE D'UNE INTERACTION ENTRE UN OPÉRATEUR ET UNE MACHINE

(30) Priority: 24.03.2020 IT 202000006238
(43) Date of publication of application: 08.02.2023
(73) Proprietor: LWT3 S.r.l., 20135 Milano (IT)
(72) Inventor: BELLUCO, Paolo, 20133 Milano (IT)
(74) Representative: Pizzoli, Antonio Mario
(86) International application number: PCT/IB2021/052385
(87) International publication number: WO 2021/191784

(56) References cited:
- WO-A1-2018/042407
- WO-A1-2019/211691
- US-A1- 2017 259 428
- US-A1- 2019 224 841

## Description

### Technical field

The present disclosure relates to a system for controlling interactions between an operator and a machine. The present description also relates to a method which can be carried out by means of said system.

### Background of the description

EP 1531726, US 2015/0148619 A1, US 6002957, US 2019/0224841 A1 and WO 2018/042407 A1 describe systems and methods that employ wearable devices for obtaining data on the efforts and movements of persons by means of sensors of different type, such as accelerometers, gyroscopes, electromyography, or vision sensors for the acquisition of motion and kinematics of the body.

However, in these known systems and methods the data obtained from the wearable devices do not allow you to control the interaction between an operator and a machine.

### Summary of the description

Object of the present description is therefore to provide a system and a method which are free from said drawbacks. Said object is achieved with a system and a method, the main features of which are specified in the attached claims, to be considered an integral part of the present description.

Thanks to their particular architecture, the system and the method according to the present description overcome the limits of the prior art, so as to automatically provide data linked between the efforts and movements of the operator and the task he is performing, and then also in relation with the data also coming from at least one machine that the operator is using, such as position of the movable elements, torques generated by the actuators, speed, temperatures and other physical quantities that measure the state of the machine.

Thus, the system and the method allow to warn the operator of possible dangers to his health, in real time and/or on a long-term thanks to a particular batch analysis, and also to send control data to the actuators of the machine which is used or to other operators who are present, so as to improve even the complementary movements in the interaction between several operators and several machines.

Thanks to a particular control subsystem, the system and the method allow to acquire data, perform analyses on the received data, send control data to the wearable devices of the operators and to the connected machines, such as speed and/or torques of the actuators, display the results of the data processing also in real time and to store both the data and the results of the processing. The control subsystem preferably uses artificial intelligence techniques to perform inferences on the data and for creating and updating patterns regarding the optimization of efforts, movements and postures which are used, also tailored to the individual operator, during his specific work tasks and during the interaction with specific machines.

The structure of the system also allows the acquisition, analysis and display of data concerning the user, the machine and preferably also the environment in which they are, so as to further improve the quality of life of the operator and of the production processes in which he interacts with the machine.

The system components also allow to collect and store automatic learning patterns to guide the improvement of ergonomics, safety and collaboration during various tasks that involve physical stress, such as repetitive and/or heavy assembly line work, handling of goods, hospital care and so on.

The system and the method preferably employ an access management subsystem based on blockchain technology, which allows the creation and the secure management of a register of entities (machines and operators) that have accessed, read or modified the data exchanged by all the system components.

In particular, the control subsystem allows to warn and advise the specific operator on the best type of movement in order to preserve his health and to send control data to the machines to optimize their behaviour according to the specific operator and his specific state at that time, such as posture, fatigue, muscle stress, heart rate and other parameters acquired by the wearable device or by the external sensors.

For example, a robot interacting with an operator in an assembly task could change the speed or the torques of the actuators in the joints, or change its configuration, position and orientation in the space of the environment in which it interacts with the operator, to avoid dangerous situations or favour the correct posture of the operator and the correct positioning of the assembled components according to the operator's parameters detected at that moment by his wearable device.

The wearable device comprises surface electromyographic sensors and the method comprises a particular step of processing the data obtained from these specific sensors, which analyses them in the frequency domain, in order to improve the quality of the data obtained from the wearable device.

### Brief description of the drawings

Further advantages and characteristics of the system and the method according to the present description will become evident to those skilled in the art from the following detailed description of some embodiments, to be considered non-limiting examples of the claims, with reference to the attached drawings wherein:
- Figure 1 shows an interaction between operator and machine;
- Figure 2 is a scheme of an embodiment of the system;
- Figure 3 is a flowchart of an embodiment of the method;
- Figure 4 is a flowchart of a variation of the method;
- Figure 5 shows a data processing step in the method.

### Exemplary embodiments

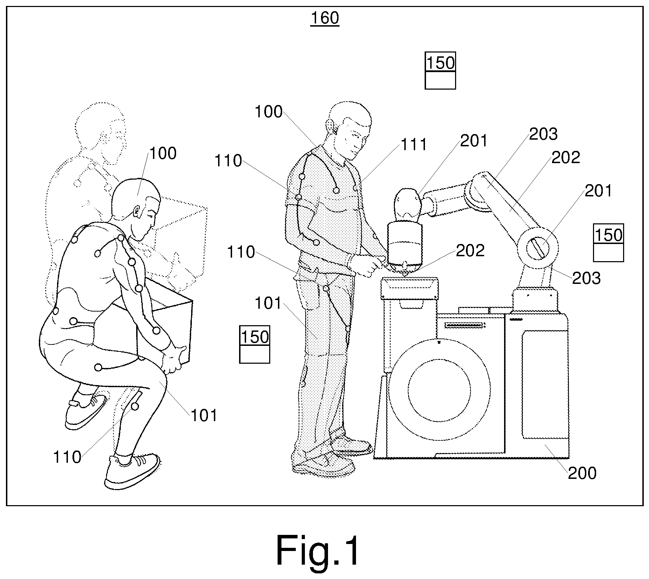

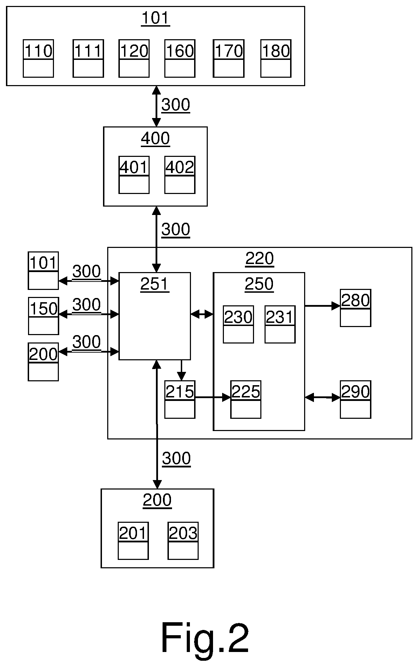

As shown in figures 1 and 2, at least one operator 100, for example a human being, may wear at least one wearable device 101, for example a suit, which is provided with one or more surface electromyographic sensors 110 suitable to measure the muscular activity of the operator 100 during his interaction with at least one machine 200, for example a robot. The machine 200 is be provided with one or more actuators 201, for example electric or hydraulic motors, and with one or more moving elements 202, for example the working head and/or arm portions of the robot, that are driven by actuators 201. The machine 200 further comprises one or more sensors 203 suitable for detecting parameters or actions of the actuators 201 and/or of the moving elements 202. The sensors 203 may comprise sensors of position, torque, speed, temperature or other physical quantities of the machine 200, in particular of the actuators 201 and the moving elements 202.

The wearable device 101 may comprise one or more further sensors 111 suitable to measure further parameters or actions of the operator 100, for example heart rate, body temperature, blood pressure, respiration, sweating, as well as posture, speed and/or accelerations of the body or of the limbs of the operator 100. The further sensors 111 may include in particular inertial measurement sensors (IMU), accelerometers, gyroscopes, magnetometers, optical fibres, motion-tracking systems based both on active or passive markers and on structured light or flight-time cameras, thermographic cameras, sensors of temperature, humidity, sound frequency and intensity, intensity of electromagnetic radiation, biometric sensors of heart rate, galvanic skin response, hydration, blood glucose, lactic acid and other biosensors, step counters and/or GPS-based geolocation systems, cellular networks (3G, 4G, 5G and any subsequent ones) radio frequency (Bluetooth, Wi-Fi, NFC, LoRaWan or others) and/or optical systems. The wearable device 101 may further comprise at least one data acquisition system 120 suitable to receive and aggregate the data received from the sensors 110, 111, at least one data processing system 170 suitable to process the data received from the sensors 110, 111, at least one data storage system 160 suitable to store the data received from the sensors 110, 111 and/or processed by the data processing system 170, as well as at least one data display system 180 suitable to display the data received from the sensors 110, 111 and/or processed by the data processing system 170.

One or more external sensors 150, such as for example pressure, sound, light, temperature or movement sensors, may be arranged in an environment 160 outside the machine 200 to detect movements of the operator 100, the machine 200 or environmental conditions, as for example environmental noise, vibrations, air temperature or the lighting in the environment 160.

A further operator 100 provided with a further wearable device 101 may be present in the same environment 160 where the first operator 100 and the machine 200 are present. Further machines 200 provided with actuators 201 and sensors 203 may also be present in the environment 160.

The system comprises at least one control subsystem 220 suitable to manage the data received or transmitted by at least one wearable device 101 and by the sensors 203 of at least one machine 200, as well as by further wearable devices 101, further sensors 203 and/or the external sensors 150, if present. The control subsystem 220 comprises in particular a data hub module 251 suitable to acquire and aggregate data transmitted by the wearable devices 101, by the sensors 203 of the machines 200 and/or by sensors external 150, as well as at least one expert system 250 suitable to analyse the data acquired by the data hub module 251, in particular by means of a real-time analysis component 230 and/or a batch analysis component 225.

The real-time analysis component 230 of the expert system 250 analyses the received data in real time and generates real-time control data, in particular warnings, alarms, information, instructions and/or commands, based on at least one interaction pattern between operator and machine, previously elaborated. Control data is sent by the expert system 250 of the control subsystem 220 to the wearable device 101 to send alerts and signals to the operator 100 through the data display system 180 and to the actuators 203 of the machine 200; and/or to the external sensors 150 monitoring the environment 160, for controlling the actions of the operator 100 and/or the machine 200.

In particular, the batch analysis component 225 employs artificial intelligence techniques (machine learning tools, such as deep artificial neural networks, pattern recognition techniques, and other known methods of supervised, unsupervised or mixed learning) which, by using the received data and interaction patterns between operator and machine stored in the control subsystem 220, allow to process control data for the wearable device 101 and/or the machine 200.

The expert system 250 may also include a sending system 231 suitable to send said control data to the wearable device 101 and/or to the machine 200 through the data hub module 251.

The control subsystem 220 may also comprise at least one data storage system 215 capable of storing the received data and the results of the processing of these data, and/or at least one data display system 280 suitable to display said data and/or results.

The data display system 180, 280 of the wearable device 101 and/or of the control subsystem 220 is suitable to output data in the format of text, tables, charts, diagrams, images, sounds and/or vibrations, and includes one or more devices suitable to represent these formats, such as for example desktop computer, laptop, tablet, handheld PC, smartphone, smartwatch, smartglasses, wearable computer, embedded systems or other similar electronic devices including output means.

The control subsystem 220 may also comprise a knowledge base 290 suitable to store the interaction patterns between operator and machine which have been generated and used by the expert system 250 to generate the control data.

The system may comprise at least one access management subsystem 400 suitable to manage access, reading and editing of the data acquired by the wearable device 101, by the machine 200 and/or by the control subsystem 220. The access management subsystem 400 may be arranged at various points in the environment 160, also on a wearable device 101 or on the machine 200, or elsewhere, and/or preferably employs a blockchain technology comprising a blockchain register 401 of the operators 100 and the machines 200 that have accessed, read or modified the data transmitted and received, and/or an access management component 402 to manage the accesses to the system on the basis of the blockchain register 401. The blockchain register 401 is in particular a distributed register that is based on blockchain technology and contains a shared and immutable data structure, wherein the register data are grouped into blocks and concatenated in chronological order. The integrity of said concatenation and of the blockchain register 401 is guaranteed by cryptographic algorithms.

The system comprises one or more of apparatuses 300 suitable to transmit data to and/or from the wearable devices 101, the external sensors 150, the machine 200, the control subsystem 220 and/or the access management subsystem 400. The apparatuses 300 may comprise wired or wireless connections, in particular local data networks, geographic data networks or cloud platforms.

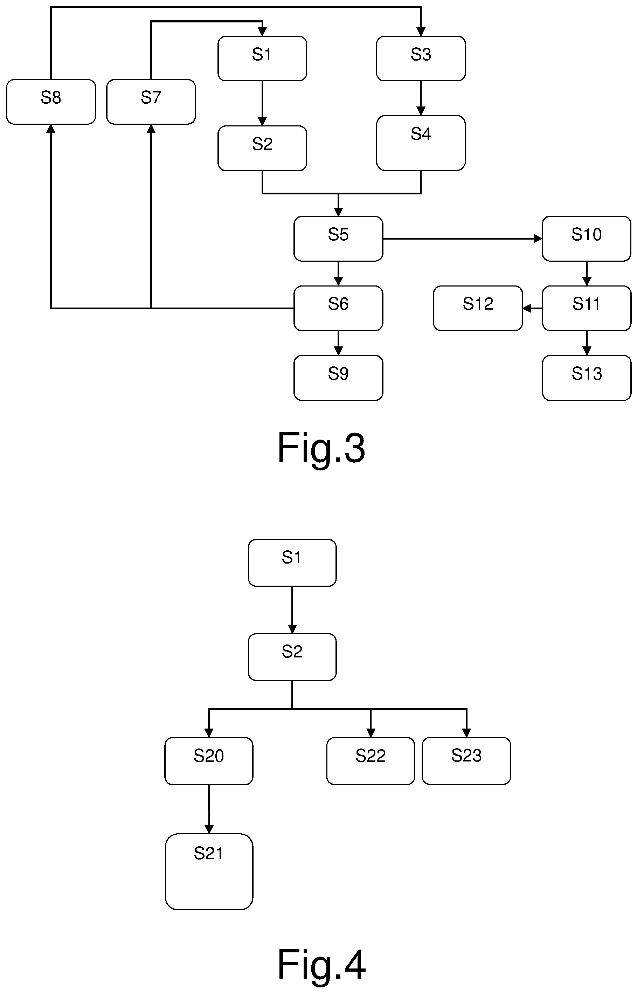

Figure 3 shows a first step S1 of the method according to an embodiment, in which the operator 100 interacts with the machine 200 and a second step S2 in which the sensors 110 and 111 of the wearable device 101 of the operator 100 transmit data to the control subsystem 220. In the meantime, the machine 200 in a step S3 is operating with the actuators 201 and the moving elements 202, so that in a step S4 the sensors 203 of the machine 200 and any external sensors 150 in the environment 160 transmit data to the control subsystem 220.

In a step S5 the data hub module 251 captures and aggregates data transmitted by the wearable device 101, by the machine 200 and/or by the external sensors 150, after which in a step S6 the real-time analysis component 230 analyses the received data and generates control data which are sent in real time through the sending system 231 and the data hub module 251 to the wearable device 101 in a step S7 and/or to the actuators 201 of the machine 200 in a step S8. Information corresponding to this control data and/or other results obtained by the real-time analysis component 230 may be displayed by the data display system 280 in a step S9.

The data managed by the data hub module 251 can be stored in the data storage system 215 in a step S10, so that they can then be analysed by the batch analysis component 225 in a step S11 to update the knowledge base 290 in a step S12 and/or to create a report in a step S13.

Figure 4 shows a first step S1 and a second step S2 of the method according to a variation of the above-mentioned embodiment, which steps correspond to those described above. However, in a step S20 the data obtained by the sensors 110, 111 are processed by the data processing system 170, which sends control data to the operator 100 by means of the data display system 180 in a step S21. In a step S22 the data obtained by the sensors 110, 111 can be stored in the data storage system 160 of the wearable device 101. In a step S23 the data obtained by the sensors 110, 111 can be sent to the data hub module 251 to carry out the steps of the method described above, starting from step S5.

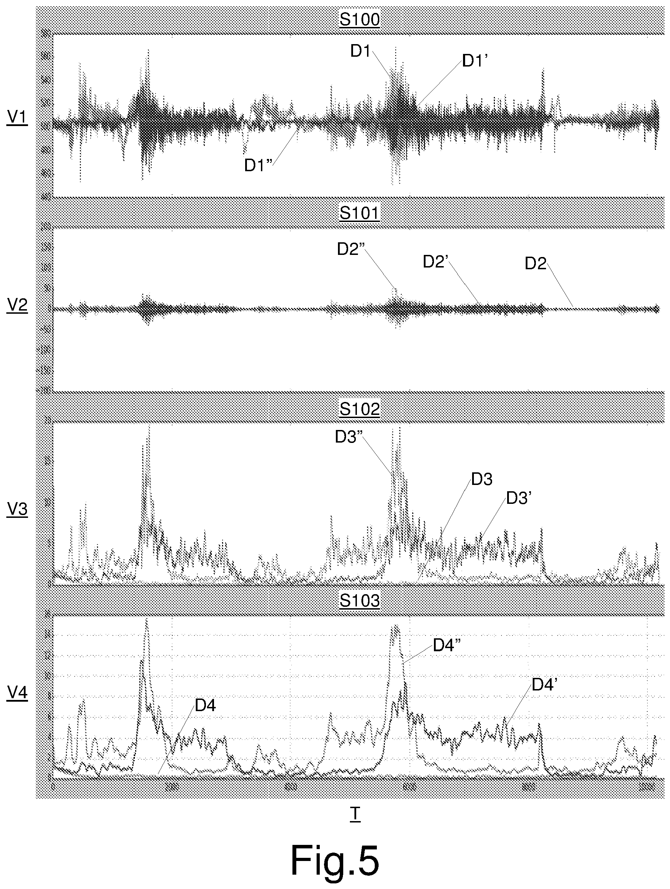

Figure 5 shows the results of the data analysis steps obtained by three electromyographic sensors 110 of three different muscle groups of the operator 100.

In a first step S100 carried out in a period of time T indicated in milliseconds, in particular during the aforementioned step S6, the raw data D1 (light grey), D1' (dark grey) and D1" (medium grey) of three sensors 110 are acquired simultaneously by the real-time analysis component 230. The values V1 of these raw data D1, D1', D1" can for example correspond to the voltage in mV or to the current in mA of an electrical signal generated by each sensor 110.

In a second step S101 the raw data D1, D1' and D1" are filtered by the real-time analysis component 230 to obtain filtered data D2, D2' and D2" with values V2. Filtering is for example obtained with a bandpass filter, in particular a third order Butterworth filter (lowcut 40, highcut 134,9).

In a third step S102 the filtered data D2, D2' and D2" are smoothed by the real-time analysis component 230, for example by means of a Savitzky-Golay filter, to obtain smoothed data D3, D3' and D3" with values V3.

In a fourth step S103 the smoothed data D3, D3' and D3" are processed by the real-time analysis component 230 to obtain root mean square values D4, D4' and D4" with values V4, which are then used for further data processing in the system and method according to the present description.

Variations or additions may be made by those skilled in the art to the embodiments described and illustrated here, while remaining within the scope of the following claims. In particular, further embodiments may comprise the technical features of one of the following claims with the addition of one or more technical features described in the text or illustrated in the drawings, taken individually or in any mutual combination.

## Claims

1. System for controlling at least one interaction between at least one operator (100) and at least one machine (200) provided with one or more actuators (201) and with one or more moving elements (202) that are driven by actuators (201), wherein the actions of the actuators (201) can be controlled by means of control data, wherein said system comprises at least one wearable device (101) which:
a. is suitable to be worn by the operator (100),
b. is provided with one or more sensors (110, 111) comprising one or more surface electromyographic sensors (110) suitable to measure the muscular activity of the operator (100) during his interaction with the machine (200),
c. comprises at least one data acquisition system (120) suitable to receive and aggregate the data received from the sensors (110, 111),
d. comprises at least one data processing system (170) suitable to process the data received from the sensors (110, 111),
e. comprises at least one data storage system (160) suitable to store the data received from the sensors (110, 111) and/or processed by the data processing system (170), and
f. comprises at least one data display system (180) suitable to display the data received by the sensors (110, 111) and/or processed by the data processing system (170),
wherein the system further comprises:
g. one or more sensors (203) suitable to detect parameters or actions of one or more of said actuators (201) of the machine (200) and/or of one or more of said moving elements (202) driven by the actuators (201),
h. at least one control subsystem (220) suitable to manage the data received from the sensors (110, 111, 203) of the wearable device (101) and of the machine (200), wherein the control subsystem (220) comprises:
h1. at least one data hub module (251) suitable to acquire and aggregate the received data, and
h2. at least one data storage system (215) suitable to store the received data and the results of the processing of these data,
**characterized in that** the control subsystem (220) also comprises at least one expert system (250) suitable to analyse the data acquired and aggregated by the data hub module (251) to generate control data according to at least one interaction pattern between operator (100) and machine (200), which interaction pattern is stored in the control subsystem (220), wherein the control subsystem (220) is suitable to send said control data to the data display system (180) of the wearable device (101) and to the actuator(s) (203) of the machine (200) to control actions of the operator (100) and of the machine (200).

2. System according to the preceding claim, wherein the wearable device (101) comprises one or more sensors (111) which are suitable to measure parameters or actions of the operator (100) and are chosen among: inertial measurement sensors (IMU), accelerometers, gyroscopes, magnetometers, optical fibres, motion-tracking systems based both on active or passive markers and on structured light or flight-time cameras, thermographic cameras, sensors of temperature, humidity, sound frequency and intensity, intensity of electromagnetic radiation, biometric sensors of heart rate, galvanic skin response, hydration, blood glucose, lactic acid and other biosensors, step counters and/or GPS-based geolocation systems, cellular networks (3G, 4G, 5G and any subsequent ones) radio frequency (Bluetooth, Wi-Fi, NFC, LoRaWan or others) and/or optical systems.

3. System according to one of the preceding claims, wherein the control subsystem (220) also comprises at least one data display system (280) suitable to display said data and/or said results.

4. System according to one of the preceding claims, wherein the data display system (180, 280) of the wearable device (101) and/or of the control subsystem (220) comprises one or more devices suitable to output data in the format of text, tables, graphs, diagrams, images, sounds and/or vibrations.

5. System according to one of the preceding claims, wherein the expert system (250) comprises a real-time analysis component (230) and/or a batch analysis component (225).

6. System according to the preceding claim, wherein the batch analysis component (225) is suitable to process with artificial intelligence techniques the received data and interaction patterns between operator (100) and machine (200) stored in the control subsystem (220) to generate the control data for the wearable device (101) and/or to the machine (200).

7. System according to one of the preceding claims, wherein the control subsystem (220) also comprises a knowledge base (290) suitable to store interaction patterns between operator (100) and machine (200), which have been generated and used by the expert system (250) to generate the control data.

8. System according to one of the preceding claims, wherein the system comprises at least one access management subsystem (400) suitable to manage access, reading and modification of the data acquired by the wearable device (101), by the machine (200) and/or by the control subsystem (220), wherein the access management subsystem (400) comprises a blockchain register (401) of one or more operators (100) and of one or more machines (200) that have accessed, read or modified the data transmitted and received, as well as an access management component (402) to manage accesses to the system according to the blockchain register (401).

9. System according to one of the preceding claims, wherein the system comprises one or more external sensors (150) suitable to be arranged in an environment (160) outside the machine (200) to detect movements of the operator (100), the machine (200) and/or conditions in the environment (160), wherein the control subsystem (220) is suitable to manage also the data received from the external sensors (150) for generating the control data for the wearable device (101) and/or for the machine (200).

10. Method for controlling at least one interaction between at least one operator (100) and at least one machine (200) provided with one or more actuators (201) and with one or more moving elements (202) that are driven by actuators (201), wherein the actions of the actuators (201) can be controlled by means of control data, which comprises the following steps:
- one or more sensors (203), suitable to detect parameters or actions of one or more of said actuators (201) of the machine (200) and/or of one or more of said moving elements (202) driven by the actuators (201), are applied to the machine (200);
- the operator (100) wears a wearable device (101) which is provided with one or more sensors (110, 111) comprising one or more surface electromyographic sensors (110) suitable to measure the muscular activity of the operator (100) during his interaction with the machine (200), wherein the wearable device (101) comprises at least one data acquisition system (120) suitable to receive and aggregate the data received from the sensors (110, 111), at least one data processing system (170) suitable to process the data received from the sensors (110, 111), at least one data storage system (160) suitable to store the data received from the sensors (110, 111) and/or processed by the data processing system (170), as well as at least one data display system (180) suitable to display the data received from the sensors (110, 111) and/or processed by the data processing system (170);
- the sensors (110, 111, 203) of the wearable device (101) and of the machine (200) transmit data to a control subsystem (220) which receives and manages this data, wherein the control subsystem (220) includes a data hub module (251) suitable to acquire and aggregate the data received, a data storage system (215) suitable to store at least the data received, as well as at least one expert system (250) suitable to analyse the data acquired and aggregated by the data hub module (251);
- the control subsystem (220) generates control data according to at least one interaction pattern between operator (100) and machine (200), which interaction pattern is stored in the control subsystem (220);
- the control subsystem (220) sends said control data to the data display system (180) of the wearable device (101) and to the actuator(s) (203) of the machine (200) to control actions of the operator (100) and of the machine (200).

11. Method according to the preceding claim, wherein the expert system (250) processes with artificial intelligence techniques the received data and interaction patterns between operator (100) and machine (200) stored in the control subsystem (220) to generate the control data for the wearable device (101) and/or for the machine (200).

12. Method according to claim 10 or 11, which comprises the following further steps:
- one or more external sensors (150) are arranged in an environment (160) outside the machine (200) to detect movements of the operator (100), the machine (200) and/or conditions of the environment (160);
- the control subsystem (220) manages also the data received from the external sensors (150);
- the control subsystem (220) generates the control data also according to the data received from the external sensors (150).

13. Method according to one of claims 10 to 12, wherein at least one access management subsystem (400) manages access, reading and modification of the data acquired by the wearable device (101), by the machine (200) and/or by the control subsystem (220), wherein the access management subsystem (400) comprises at least one blockchain register (401) of one or more operators (100) and of one or more machines (200) that have accessed, read or modified the data transmitted and received, as well as at least one access management component (402) to manage accesses to the system according to the blockchain register (401).

14. Method according to one of claims 10 to 13, wherein the analysis of the data of the surface electromyographic sensors (110) comprises the following steps:
- the raw data (D1, D1', D1") of the sensors (110) are filtered in the frequency domain to obtain filtered data (D2, D2', D2");
- said filtered data (D2, D2', D2") are smoothed;
- said smoothed data (D3, D3', D3") are processed to obtain root mean square values (D4, D4', D4") of the data.

15. Method according to one of claims 10 to 14, wherein the steps are carried out by a system according to one of claims 1 to 9.

## Patentansprüche

1. System zur Steuerung mindestens einer Interaktion zwischen mindestens einem Bediener (100) und mindestens einer Maschine (200), die mit einem oder mehreren Aktuatoren (201) und mit einem oder mehreren beweglichen Elementen (202) versehen ist, die von Aktuatoren (201) angetrieben werden, wobei die Aktionen der Aktuatoren (201) mittels Steuerungsdaten gesteuert werden können, wobei das System mindestens eine tragbare Vorrichtung (101) umfasst, die:
a. dazu geeignet ist, von dem Bediener (100) getragen zu werden,
b. mit einem oder mehreren Sensoren (110, 111) versehen ist, die einen oder mehrere elektromyografische Oberflächensensoren (110) umfassen, die zum Messen der Muskelaktivität des Bedieners (100) während seiner Interaktion mit der Maschine (200) geeignet sind,
c. mindestens ein Datenerfassungssystem (120) umfasst, das zum Empfangen und Aggregieren der von den Sensoren (110, 111) empfangenen Daten geeignet ist,
d. mindestens ein Datenverarbeitungssystem (170) umfasst, das zum Verarbeiten der von den Sensoren (110, 111) empfangenen Daten geeignet ist,
e. mindestens ein Datenspeichersystem (160) umfasst, das zum Speichern der von den Sensoren (110, 111) empfangenen und/oder von dem Datenverarbeitungssystem (170) verarbeiteten Daten geeignet ist, und
f. mindestens ein Datenanzeigesystem (180) umfasst, das zum Anzeigen der von den Sensoren (110, 111) empfangenen und/oder von dem Datenverarbeitungssystem (170) verarbeiteten Daten geeignet ist,
wobei das System ferner umfasst:
g. einen oder mehrere Sensoren (203), die zum Erfassen von Parametern oder Aktionen eines oder mehrerer der Aktuatoren (201) der Maschine (200) und/oder eines oder mehrerer der von den Aktuatoren (201) angetriebenen beweglichen Elemente (202) geeignet sind,
h. mindestens ein Steuerungsuntersystem (220), das zum Verwalten der von den Sensoren (110, 111, 203) der tragbaren Vorrichtung (101) und der Maschine (200) empfangenen Daten geeignet ist, wobei das Steuerungsuntersystem (220) umfasst:
h1. mindestens ein Datenhubmodul (251), das zum Erfassen und Aggregieren der empfangenen Daten geeignet ist, und
h2. mindestens ein Datenspeichersystem (215), das zum Speichern der empfangenen Daten und der Ergebnisse der Verarbeitung dieser Daten geeignet ist,
**dadurch gekennzeichnet, dass** das Steuerungsuntersystem (220) außerdem mindestens ein Expertensystem (250) umfasst, das zum Analysieren der von dem Datenhubmodul (251) erfassten und aggregierten Daten geeignet ist, um Steuerungsdaten entsprechend dem mindestens einen Interaktionsmuster zwischen Bediener (100) und Maschine (200) zu erzeugen, wobei das Interaktionsmuster in dem Steuerungsuntersystem (220) gespeichert ist, wobei das Steuerungsuntersystem (220) zum Senden der Steuerungsdaten an das Datenanzeigesystem (180) der tragbaren Vorrichtung (101) und an den/die Aktuator(en) (203) der Maschine (200) geeignet ist, um die Aktionen des Bedieners (100) und der Maschine (200) zu steuern.

2. System nach dem vorhergehenden Anspruch, wobei die tragbare Vorrichtung (101) einen oder mehrere Sensoren (111) umfasst, die zum Messen von Parametern oder Aktionen des Bedieners (100) geeignet sind und aus folgenden ausgewählt werden: Trägheitsmesssensoren (IMU), Beschleunigungsmessern, Gyroskopen, Magnetometern, optischen Fasern, Bewegungserfassungssystemen, die sowohl auf aktiven oder passiven Markern als auch auf strukturiertem Licht oder Flugzeitkameras basieren, Wärmebildkameras, Sensoren für Temperatur, Feuchtigkeit, Schallfrequenz und -intensität, Intensität elektromagnetischer Strahlung, biometrischen Sensoren für Herzfrequenz, galvanische Hautreaktion, Hydratation, Blutzucker, Milchsäure und anderen Biosensoren, Schrittzählern und/oder GPS-basierte Geolokalisierungssystemen, Mobilfunknetzen (3G, 4G, 5G und alle nachfolgenden), Radiofrequenz (Bluetooth, Wi-Fi, NFC, LoRaWan oder andere) und/oder optischen Systemen.

3. System nach einem der vorhergehenden Ansprüche, wobei das Steuerungsuntersystem (220) auch mindestens ein Datenanzeigesystem (280) umfasst, das zum Anzeigen der Daten und/oder der Ergebnisse geeignet ist.

4. System nach einem der vorhergehenden Ansprüche, wobei das Datenanzeigesystem (180, 280) der tragbaren Vorrichtung (101) und/oder des Steuerungsuntersystems (220) eine oder mehrere Vorrichtungen umfasst, die zum Ausgeben von Daten im Format von Text, Tabellen, Grafiken, Diagrammen, Bildern, Tönen und/oder Vibrationen geeignet sind.

5. System nach einem der vorhergehenden Ansprüche, wobei das Expertensystem (250) eine Echtzeitanalysekomponente (230) und/oder eine Chargenanalysekomponente (225) umfasst.

6. System nach dem vorhergehenden Anspruch, wobei die Chargenanalysekomponente (225) zum Verarbeiten der empfangenen Daten und Interaktionsmuster zwischen Bediener (100) und Maschine (200), die in dem Steuerungsuntersystem (220) gespeichert sind, mit Techniken der künstlichen Intelligenz geeignet ist, um die Steuerungsdaten für die tragbare Vorrichtung (101) und/oder die Maschine (200) zu erzeugen.

7. System nach einem der vorhergehenden Ansprüche, wobei das Steuerungsuntersystem (220) auch eine Wissensbasis (290) umfasst, die zum Speichern von Interaktionsmustern zwischen Bediener (100) und Maschine (200) geeignet ist, die von dem Expertensystem (250) zur Erzeugung der Steuerungsdaten erzeugt und verwendet wurden.

8. System nach einem der vorhergehenden Ansprüche, wobei das System mindestens ein Zugriffsverwaltungsuntersystem (400) umfasst, das zum Verwalten des Zugriffs, des Lesens und der Änderung der von der tragbaren Vorrichtung (101), von der Maschine (200) und/oder von dem Steuerungsuntersystem (220) erfassten Daten geeignet ist, wobei das Zugriffsverwaltungsuntersystem (400) ein Blockchain-Register (401) eines oder mehrerer Bediener (100) und einer oder mehrerer Maschinen (200), die auf die übertragenen und empfangenen Daten zugegriffen, diese gelesen oder geändert haben, sowie eine Zugriffsverwaltungskomponente (402) zum Verwalten der Zugriffe auf das System entsprechend dem Blockchain-Register (401) umfasst.

9. System nach einem der vorhergehenden Ansprüche, wobei das System einen oder mehrere externe Sensoren (150) umfasst, die zum Anordnen in einer Umgebung (160) außerhalb der Maschine (200) geeignet sind, um Bewegungen des Bedieners (100), der Maschine (200) und/oder Bedingungen in der Umgebung (160) zu erfassen, wobei das Steuerungsuntersystem (220) auch zum Verwalten der von den externen Sensoren (150) empfangenen Daten geeignet ist, um die Steuerungsdaten für die tragbare Vorrichtung (101) und/oder für die Maschine (200) zu erzeugen.

10. Verfahren zur Steuerung mindestens einer Interaktion zwischen mindestens einem Bediener (100) und mindestens einer Maschine (200), die mit einem oder mehreren Aktuatoren (201) und mit einem oder mehreren beweglichen Elementen (202) versehen ist, die von Aktuatoren (201) angetrieben werden, wobei die Aktionen der Aktuatoren (201) mittels Steuerungsdaten gesteuert werden können, das die folgenden Schritte umfasst:
- ein oder mehrere Sensoren (203), die zum Erfassen von Parametern oder Aktionen eines oder mehrerer der Aktuatoren (201) der Maschine (200) und/oder eines oder mehrerer der von den Aktuatoren (201) angetriebenen beweglichen Elemente (202) geeignet sind, werden an der Maschine (200) angebracht;
- der Bediener (100) trägt eine tragbare Vorrichtung (101), die mit einem oder mehreren Sensoren (110, 111) versehen ist, die einen oder mehrere elektromyografische Oberflächensensoren (110) umfassen, die zum Messen der Muskelaktivität des Bedieners (100) während seiner Interaktion mit der Maschine (200) geeignet sind, wobei die tragbare Vorrichtung (101) mindestens ein Datenerfassungssystem (120), das zum Empfangen und Aggregieren der von den Sensoren (110, 111) empfangenen Daten geeignet ist, mindestens ein Datenverarbeitungssystem (170), das zum Verarbeiten der von den Sensoren (110, 111) empfangenen Daten geeignet ist, mindestens ein Datenspeichersystem (160), das zum Speichern der von den Sensoren (110, 111) empfangenen und/oder von dem Datenverarbeitungssystem (170) verarbeiteten Daten geeignet ist, sowie mindestens ein Datenanzeigesystem (180) umfasst, das zum Anzeigen der von den Sensoren (110, 111) empfangenen und/oder von dem Datenverarbeitungssystem (170) verarbeiteten Daten geeignet ist;
- die Sensoren (110, 111, 203) der tragbaren Vorrichtung (101) und der Maschine (200) übertragen Daten an ein Steuerungsuntersystem (220), das diese Daten empfängt und verwaltet, wobei das Steuerungsuntersystem (220) ein Datenhubmodul (251), das zum Erfassen und Aggregieren der empfangenen Daten geeignet ist, ein Datenspeichersystem (215), das zum Speichern mindestens der empfangenen Daten geeignet ist, sowie mindestens ein Expertensystem (250) umfasst, das zum Analysieren der von dem Datenhubmodul (251) erfassten und aggregierten Daten geeignet ist;
- das Steuerungsuntersystem (220) erzeugt Steuerungsdaten entsprechend mindestens einem Interaktionsmuster zwischen Bediener (100) und Maschine (200), wobei das Interaktionsmuster in dem Steuerungsuntersystem (220) gespeichert ist;
- das Steuerungsuntersystem (220) sendet die Steuerungsdaten an das Datenanzeigesystem (180) der tragbaren Vorrichtung (101) und an den/die Aktuator(en) (203) der Maschine (200), um die Aktionen des Bedieners (100) und der Maschine (200) zu steuern.

11. Verfahren nach dem vorhergehenden Anspruch, wobei das Expertensystem (250) die empfangenen Daten und Interaktionsmuster zwischen Bediener (100) und Maschine (200), die in dem Steuerungsuntersystem (220) gespeichert sind, mit Techniken der künstlichen Intelligenz verarbeitet, um die Steuerungsdaten für die tragbare Vorrichtung (101) und/oder die Maschine (200) zu erzeugen.

12. Verfahren nach Anspruch 10 oder 11, das die folgenden weiteren Schritte umfasst:
- ein oder mehrere externe Sensoren (150) sind in einer Umgebung (160) außerhalb der Maschine (200) angeordnet, um Bewegungen des Bedieners (100), der Maschine (200) und/oder Bedingungen der Umgebung (160) zu erfassen;
- das Steuerungsuntersystem (220) verwaltet auch die von den externen Sensoren (150) empfangenen Daten;
- das Steuerungsuntersystem (220) erzeugt die Steuerungsdaten auch entsprechend den von den externen Sensoren (150) empfangenen Daten.

13. Verfahren nach einem der Ansprüche 10 bis 12, wobei mindestens ein Zugriffsverwaltungsuntersystem (400) den Zugriff, das Lesen und die Änderung der von der tragbaren Vorrichtung (101), von der Maschine (200) und/oder von dem Steuerungsuntersystem (220) erfassten Daten verwaltet, wobei das Zugriffsverwaltungsuntersystem (400) mindestens ein Blockchain-Register (401) eines oder mehrerer Bediener (100) und einer oder mehrerer Maschinen (200), die auf die übertragenen und empfangenen Daten zugegriffen, diese gelesen oder geändert haben, sowie mindestens eine Zugriffsverwaltungskomponente (402) zum Verwalten der Zugriffe auf das System entsprechend dem Blockchain-Register (401) umfasst.

14. Verfahren nach einem der Ansprüche 10 bis 13, wobei die Analyse der Daten der elektromyografischen Oberflächensensoren (110) die folgenden Schritte umfasst:
- die Rohdaten (D1, D1', D1") der Sensoren (110) werden im Frequenzbereich gefiltert, um gefilterte Daten (D2, D2', D2") zu erhalten;
- die gefilterten Daten (D2, D2', D2") werden geglättet;
- die geglätteten Daten (D3, D3', D3") werden verarbeitet, um quadratische Mittelwerte (D4, D4', D4") der Daten zu erhalten.

15. Verfahren nach einem der Ansprüche 10 bis 14, wobei die Schritte von einem System nach einem der Ansprüche 1 bis 9 durchgeführt werden.

## Revendications

1. Système de commande d'au moins une interaction entre au moins un opérateur (100) et au moins une machine (200) munie d'un ou plusieurs actionneurs (201) et d'un ou plusieurs éléments mobiles (202) qui sont entraînés par des actionneurs (201), dans lequel les actions des actionneurs (201) peuvent être commandées au moyen de données de commande, dans lequel ledit système comprend au moins un dispositif portable (101) qui :
a. convient au port par l'opérateur (100),
b. est muni d'un ou plusieurs capteurs (110, 111) comprenant un ou plusieurs capteurs électromyographiques de surface (110) adaptés pour mesurer l'activité musculaire de l'opérateur (100) lors de l'interaction de celui-ci avec la machine (200),
c. comprend au moins un système d'acquisition de données (120) adapté pour recevoir et agréger les données reçues des capteurs (110, 111),
d. comprend au moins un système de traitement de données (170) adapté pour traiter les données reçues des capteurs (110, 111),
e. comprend au moins un système de stockage de données (160) adapté pour stocker les données reçues des capteurs (110, 111) et/ou traitées par le système de traitement de données (170), et
f. comprend au moins un système d'affichage de données (180) adapté pour afficher les données reçues par les capteurs (110, 111) et/ou traitées par le système de traitement de données (170),
dans lequel le système comprend en outre :
g. un ou plusieurs capteurs (203) appropriés pour détecter des paramètres ou des actions d'un ou plusieurs desdits actionneurs (201) de la machine (200) et/ou d'un ou plusieurs desdits éléments mobiles (202) entraînés par les actionneurs (201),
h. au moins un sous-système de commande (220) approprié pour gérer les données reçues des capteurs (110, 111, 203) du dispositif portable (101) et de la machine (200), dans lequel le sous-système de commande (220) comprend :
h1. au moins un module de concentrateur de données (251) approprié pour acquérir et agréger les données reçues, et
h2. au moins un système de stockage de données (215) adapté pour stocker les données reçues et les résultats du traitement de ces données,
**caractérisé en ce que** le sous-système de commande (220) comprend également au moins un système expert (250) approprié pour analyser les données acquises et agrégées par le module de concentrateur de données (251) pour générer des données de commande selon au moins un modèle d'interaction entre l'opérateur (100) et la machine (200), lequel modèle d'interaction est stocké dans le sous-système de commande (220), dans lequel le sous-système de commande (220) est approprié pour envoyer lesdites données de commande au système d'affichage de données (180) du dispositif portable (101) et à (aux) l'actionneur (actionneurs) (203) de la machine (200) pour commander les actions de l'opérateur (100) et de la machine (200).

2. Système selon la revendication précédente, dans lequel le dispositif portable (101) comprend un ou plusieurs capteurs (111) qui sont appropriés pour mesurer des paramètres ou des actions de l'opérateur (100) et sont choisis parmi : des capteurs de mesure inertiels (en Anglais « inertial measurement sensors » pour IMU), des accéléromètres, des gyroscopes, des magnétomètres, des fibres optiques, des systèmes de suivi de mouvement basés à la fois sur des marqueurs actifs ou passifs et sur des caméras lumineuses ou de temps de vol structurées, des caméras thermographiques, des capteurs de température, d'humidité, de fréquence et d'intensité sonores, d'intensité du rayonnement électromagnétique, des capteurs biométriques de fréquence cardiaque, de réponse galvanique de la peau, d'hydratation, de glycémie, d'acide lactique et d'autres biocapteurs, des compteurs de pas et/ou des systèmes de géolocalisation basés sur GPS, des réseaux cellulaires (3G, 4G, 5G et tous les suivants), des radiofréquences (Bluetooth, Wi-Fi, NFC, LoRaWan ou autres) et/ou des systèmes optiques.

3. Système selon l'une des revendications précédentes, dans lequel le sous-système de commande (220) comprend également au moins un système d'affichage de données (280) approprié pour afficher lesdites données et/ou lesdits résultats.

4. Système selon l'une des revendications précédentes, dans lequel le système d'affichage de données (180, 280) du dispositif portable (101) et/ou du sous-système de commande (220) comprend un ou plusieurs dispositifs appropriés pour sortir des données sous forme de texte, de tableaux, de graphiques, de diagrammes, d'images, de sons et/ou de vibrations.

5. Système selon l'une des revendications précédentes, dans lequel le système expert (250) comprend un composant d'analyse en temps réel (230) et/ou un composant d'analyse de lots (225).

6. Système selon la revendication précédente, dans lequel le composant d'analyse de lots (225) est approprié pour traiter avec des techniques d'intelligence artificielle les données reçues et les modèles d'interaction entre l'opérateur (100) et la machine (200) stockés dans le sous-système de commande (220) pour générer les données de commande pour le dispositif portable (101) et/ou pour la machine (200).

7. Système selon l'une des revendications précédentes, dans lequel le sous-système de commande (220) comprend également une base de connaissances (290) appropriée pour stocker des modèles d'interaction entre l'opérateur (100) et la machine (200), qui ont été générés et utilisés par le système expert (250) pour générer les données de commande.

8. Système selon l'une des revendications précédentes, dans lequel le système comprend au moins un sous-système de gestion d'accès (400) approprié pour gérer l'accès, la lecture et la modification des données acquises par le dispositif portable (101), par la machine (200) et/ou par le sous-système de commande (220), dans lequel le sous-système de gestion d'accès (400) comprend un registre blockchain (401) d'un ou plusieurs opérateurs (100) et d'une ou plusieurs machines (200) qui ont accédé, lu ou modifié les données transmises et reçues, ainsi qu'un composant de gestion d'accès (402) pour gérer les accès au système selon le registre blockchain (401).

9. Système selon l'une des revendications précédentes, dans lequel le système comprend un ou plusieurs capteurs externes (150) appropriés pour être disposés dans un environnement (160) à l'extérieur de la machine (200) pour détecter les mouvements de l'opérateur (100), de la machine (200) et/ou des conditions dans l'environnement (160), dans lequel le sous-système de commande (220) est approprié pour gérer également les données reçues des capteurs externes (150) pour générer les données de commande pour le dispositif portable (101) et/ou pour la machine (200).

10. Procédé de commande d'au moins une interaction entre au moins un opérateur (100) et au moins une machine (200) munie d'un ou plusieurs actionneurs (201) et d'un ou plusieurs éléments mobiles (202) qui sont entraînés par des actionneurs (201), dans lequel les actions des actionneurs (201) peuvent être commandées au moyen de données de commande, qui comprend les étapes suivantes :
- un ou plusieurs capteurs (203), appropriés pour détecter des paramètres ou des actions d'un ou plusieurs desdits actionneurs (201) de la machine (200) et/ou d'un ou plusieurs desdits éléments mobiles (202) entraînés par les actionneurs (201), sont appliqués à la machine (200) ;
- l'opérateur (100) porte un dispositif portable (101) qui est muni d'un ou plusieurs capteurs (110, 111) comprenant un ou plusieurs capteurs électromyographiques de surface (110) adaptés pour mesurer l'activité musculaire de l'opérateur (100) lors de son interaction avec la machine (200), dans lequel le dispositif portable (101) comprend au moins un système d'acquisition de données (120) adapté pour recevoir et agréger les données reçues des capteurs (110, 111), au moins un système de traitement de données (170) adapté pour traiter les données reçues des capteurs (110, 111), au moins un système de stockage de données (160) adapté pour stocker les données reçues des capteurs (110, 111) et/ou traitées par le système de traitement de données (170), ainsi qu'au moins un système d'affichage de données (180) adapté pour afficher les données reçues des capteurs (110, 111) et/ou traitées par le système de traitement de données (170) ;
- les capteurs (110, 111, 203) du dispositif portable (101) et de la machine (200) transmettent des données à un sous-système de commande (220) qui reçoit et gère ces données, dans lequel le sous-système de commande (220) inclut un module de concentrateur de données (251) adapté pour acquérir et agréger les données reçues, un système de stockage de données (215) adapté pour stocker au moins les données reçues, ainsi qu'au moins un système expert (250) adapté pour analyser les données acquises et agrégées par le module de concentrateur de données (251) ;
- le sous-système de commande (220) génère des données de commande selon au moins un modèle d'interaction entre l'opérateur (100) et la machine (200), lequel modèle d'interaction est stocké dans le sous-système de commande (220) ;
- le sous-système de commande (220) envoie lesdites données de commande au système d'affichage de données (180) du dispositif portable (101) et à (aux) l'actionneur (actionneurs) (203) de la machine (200) pour commander les actions de l'opérateur (100) et de la machine (200).

11. Procédé selon la revendication précédente, dans lequel le système expert (250) traite avec des techniques d'intelligence artificielle les données reçues et les modèles d'interaction entre l'opérateur (100) et la machine (200) stockés dans le sous-système de commande (220) pour générer les données de commande pour le dispositif portable (101) et/ou pour la machine (200).

12. Procédé selon la revendication 10 ou 11, qui comprend les étapes supplémentaires suivantes :
- un ou plusieurs capteurs externes (150) sont disposés dans un environnement (160) à l'extérieur de la machine (200) pour détecter les mouvements de l'opérateur (100), de la machine (200) et/ou des conditions de l'environnement (160) ;
- le sous-système de commande (220) gère également les données reçues des capteurs externes (150) ;
- le sous-système de commande (220) génère les données de commande également en fonction des données reçues des capteurs externes (150).

13. Procédé selon l'une des revendications 10 à 12, dans lequel au moins un sous-système de gestion d'accès (400) gère l'accès, la lecture et la modification des données acquises par le dispositif portable (101), par la machine (200) et/ou par le sous-système de commande (220), dans lequel le sous-système de gestion d'accès (400) comprend au moins un registre blockchain (401) d'un ou plusieurs opérateurs (100) et d'une ou plusieurs machines (200) qui ont accédé, lu ou modifié les données transmises et reçues, ainsi qu'au moins un composant de gestion d'accès (402) pour gérer les accès au système selon le registre blockchain (401).

14. Procédé selon l'une des revendications 10 à 13, dans lequel l'analyse des données des capteurs électromyographiques de surface (110) comprend les étapes suivantes :
- les données brutes (D1, D1', D1") des capteurs (110) sont filtrées dans le domaine fréquentiel pour obtenir des données filtrées (D2, D2', D2") ;
- lesdites données filtrées (D2, D2', D2") sont lissées ;
- lesdites données lissées (D3, D3', D3") sont traitées pour obtenir des valeurs quadratiques moyennes (D4, D4', D4") des données.

15. Procédé selon l'une des revendications 10 à 14, dans lequel les étapes sont réalisées par un système selon l'une des revendications 1 à 9.
